# NEW EUROPEAN PATENT SPECIFICATION

(11) **EP 0 419 070 B2**
(45) Date of publication and mention of the opposition decision: **03.11.1999**
(45) Mention of the grant of the patent: 13.12.1995
(21) Application number: 90309535.4
(22) Date of filing: 31.08.1990
(51) Int. Cl.: A61B 19/00

(54) **Medical three-dimensional locating apparatus**
Medizinische Vorrichtung zur dreidimensionalen Lokalisierung
Dispositif médical de localisation tridimensionnelle

(30) Priority: 20.09.1989 JP 24202789
(43) Date of publication of application: 27.03.1991
(73) Proprietor: MITAKA KOHKI CO., LTD., Mitaka-shi Tokyo (JP); Takizawa, Takaaki, Fukuyama-shi, Hiroshima-ken (JP)
(72) Inventor: Ota, Kosuke, Fukuyama-shi, Hiroshima-ken (JP); Takizawa, Takaaki, Fukuyama-shi, Hiroshima-ken (JP); Nakamura, Giichi, Mitaka-shi, Tokyo (JP); Nakamura, Katsushige, Hachioji-shi, Tokyo (JP)
(74) Representative: Gold, Tibor Z.

(56) References cited:
- EP-A- 0 286 170
- EP-A- 0 293 228
- WO-A-88/08282
- WO-A-88/09151
- CH-A- 482 439
- US-A- 4 863 133

## Description

### BACKGROUND OF THE INVENTION

### Field of the Invention

The present invention relates to a medical three-dimensional locating apparatus and, more particularly, to a medical three-dimensional locating apparatus for encephalic surgical operation.

### Description of the Prior Art

A stand mechanism for medical optical equipment is known from EP-A-0 293 228 and comprises a support stand, a central shaft rotatably mounted on the support stand, a parallel linkage connected to the central shaft, a swivel arrangement at a free end of the parallel linkage and optical equipment carried by the swivel arrangement. This apparatus is designed for optical observations.

The diffusion of CT scanning apparatus and MRI scanning apparatus has revolutionized encephalic nerve surgery, and three-dimensional imaging diagnosis for encephalic nerve-surgical operation has replaced conventional imaging diagnosis employing simple cranial photography or cerebral angiography.

Imaging diagnosis employing a CT scanning apparatus or a MRI scanning apparatus, however, is able only to determine the position of a focus, i.e., a target, three-dimensionally and is unable to reproduce the position data obtained by imaging diagnosis in the patient's head. Although various CT induction localization encephalic surgical apparatus are being developed currently for the reproduction of an optional point in a picture obtained by CT scanning in the patient's head, the accuracy of those apparatus is not necessarily satisfactory.

Even if the accuracy of reproduction of those CT induction locating encephalic surgical apparatus being developed is satisfactory, still another problem specific to encephalic surgical operation requiring craniotomy remains unsolved. That is, in craniotomy, surgical opening of a portion of the patient's skull corresponding to the focus is infeasible even if the position of the portion corresponding to the focus is determined and the position of the portion is nearest to the focus, in case particularly important nervous tissues exist between the portion of the patient's skull and the focus. In such a case, another portion of the patient's skull which will allow craniotomy without interfering with the particularly important nervous tissues, even if the position of this portion is remote from the focus, must be opened to approach the focus. Nevertheless, the conventional CT induction locating encephalic surgical apparatus requires a difficult operation to approach the focus from the remote position on the patient's skull and to find the position of an optimum portion for craniotomy.

Accordingly, it is an object of the present invention to provide a medical three-dimensional locating apparatus capable of accurately reproducing three-dimensional position data representing the position of the focus in the patient's head, obtained by CT scanning or MRI scanning in actually carrying out a surgical operation, and requiring a simple operation for finding the position of an optimum portion for craniotomy.

### SUMMARY OF THE INVENTION

The present invention provides a medical three-dimensional locating apparatus according to the features of claim 1.

Preferably, the intersection point of the first and second axis remains fixed and the extremity of the indicating unit is directed toward the intersection point of the first and second axis, even if the first arm and the second arm are turned individually. Accordingly, the extremity of the indicating unit is directed always toward the focus when the intersection point of the first and second axes is located at the focus in the patient's head by moving the arm unit by the support unit. Since the extremity of the indicating unit is directed always toward the focus regardless of the position of the indicating unit, an optimum approach angle (position for craniotomy) in which to approach the focus can readily be selected with reference to a picture obtained by CT scanning or MRI scanning.

Since the position of the intersection point of the first and second axes and the position of the extremity of the indicating unit with respect to the intersection point of the first and second axes are detected by the position detectors, the three-dimensional position data obtained by a CT scanning apparatus or a MRI scanning apparatus can accurately be reproduced in the patient's head for surgical operation by operating the arm unit through the combination of the data obtained by the position detectors and the position data of the focus obtained by CT scanning or MRI scanning, monitoring the operation of the arm unit on a computer-controlled display.

The above and other objects, features and advantages of the present invention will become more apparent from the following description taken in connection with the accompanying drawings.

### BRIEF DESCRIPTION OF THE DRAWINGS

Figure 1 is a side elevation of a medical three-dimensional locating apparatus in a preferred embodiment according to the present invention;
Figure 2 is a plan view of the medical three-dimensional locating apparatus of Fig. 1;
Figure 3 is a perspective view of an arm unit;
Figure 4 is a perspective view taken in the direction of an arrow IV in Fig. 3;
Figure 5 is an exploded perspective view of the patient's head, showing the respective positions of an origin and the focus;
Figure 6 is a sectional view of the patient's head, illustrating the relation between a mark and the origin;
Figure 7 is a sectional view of the patient's head of assistance in explaining an angular focus locating method for locating the focus;
Figure 8 is a sectional view of the patient's head of assistance in explaining a coordinate focus locating method for locating the focus; and
Figure 9 is a sectional view of the patient's head of assistance in explaining a medical three-dimensional locating apparatus in another embodiment which is not part of the present invention.

### DESCRIPTION OF THE PREFERRED EMBODIMENTS

Preferred embodiments of the present invention will be described hereinafter with reference to the accompanying drawings.

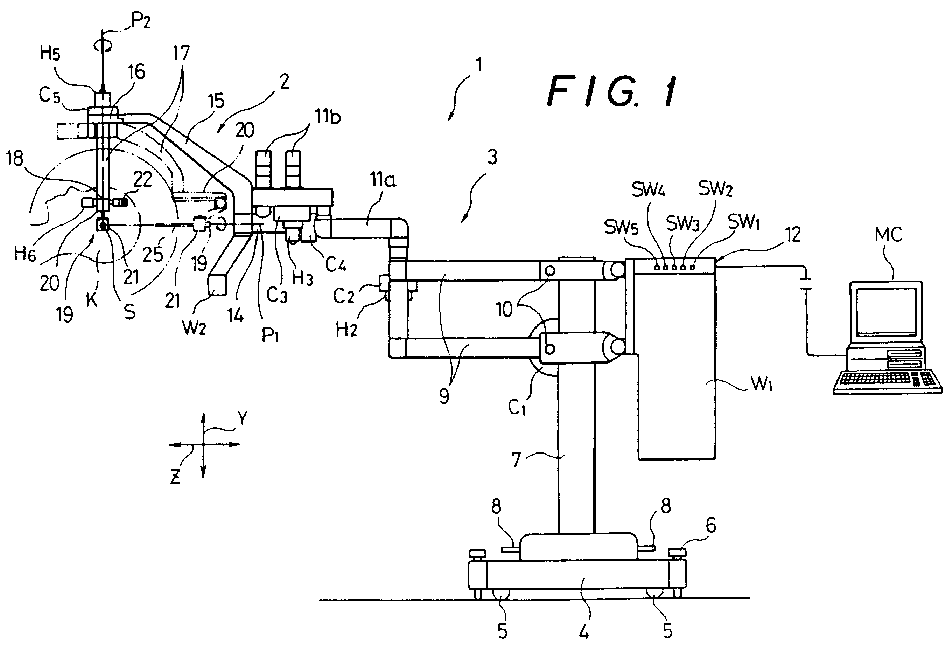

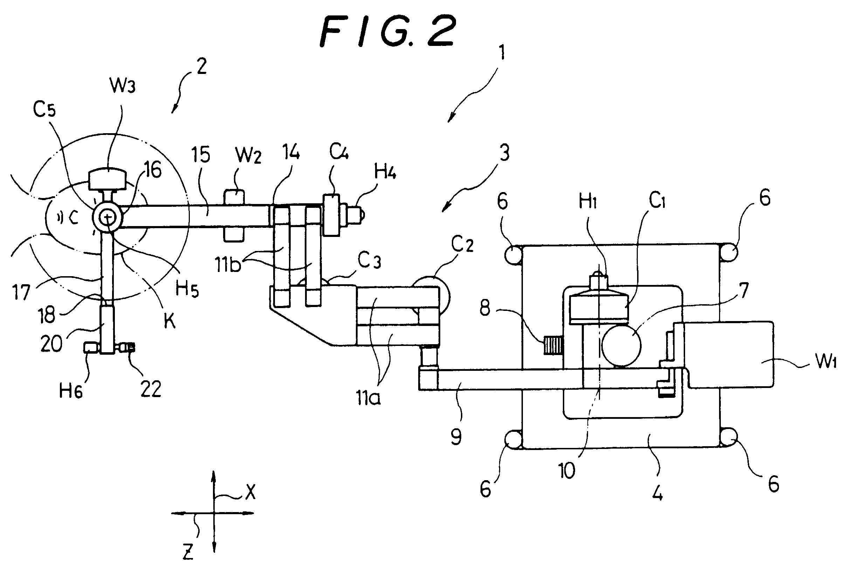

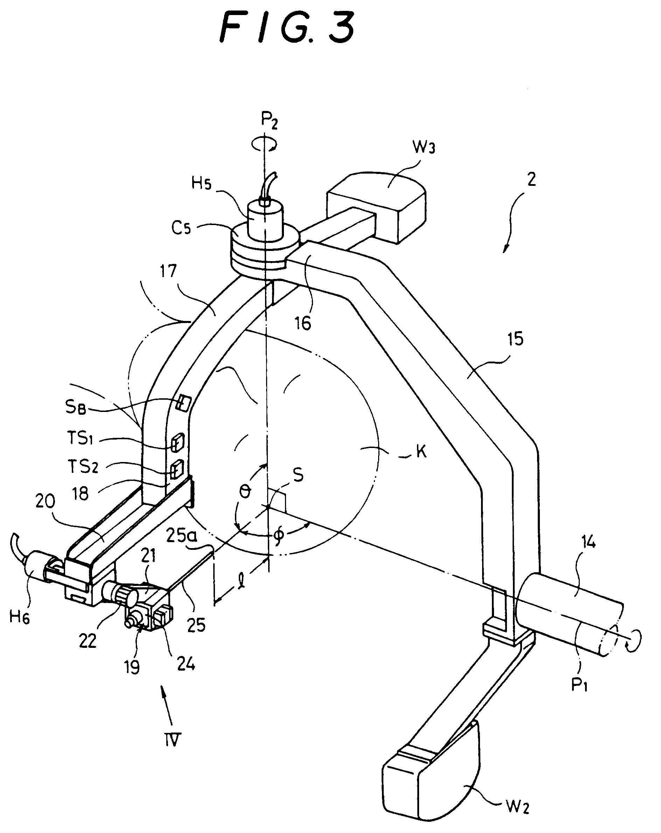

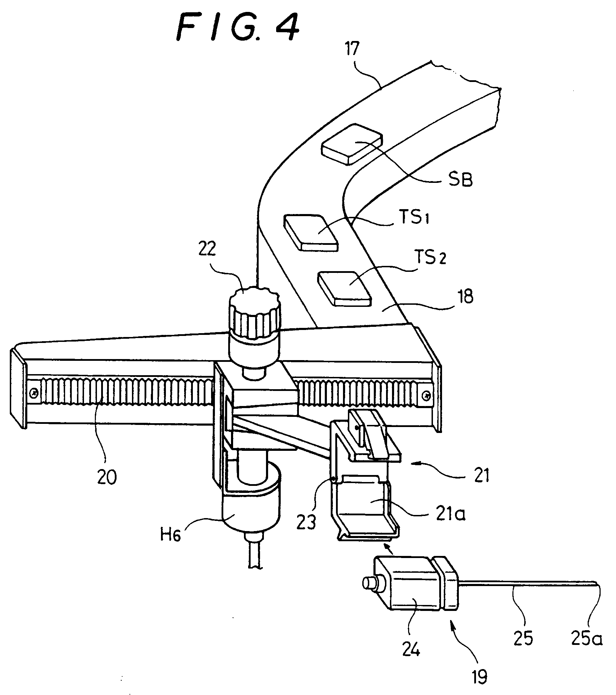

Referring to Figs. 1 to 8, a medical three-dimensional locating apparatus 1 in a preferred embodiment according to the present invention comprises an arm unit 2 to be positioned relative to the patient's head K, and a support unit 3 for supporting the arm unit 2 at an optional position. The medical three-dimensional locating apparatus 1 is controlled by a computer MC.

The support unit 3 has a base 4 provided with caster wheels 5 for free movement, and immobilizing screws 6 provided at its four corners to secure the base 4 to the floor. A post 7 is set upright in the central portion of the base 4. The post 7 can turn accurately through an angle of 180° when either of pedals 8 provided in front of and behind the post 7 is pedaled. Parallel links 9 forming a vertical-swing linkage are supported pivotally by pivots 10 on the upper portion of the post 7 for swing motion on the pivots 10 in a vertical plane to shift the support unit 3 vertically. An electromagnetic clutch C₁ and a rotary encoder H₁, i.e., a position detector, are associated with the pivots 10. The electromagnetic clutch C₁ holds the vertical-swing linkage including the links 9 in a desired position relative to the post 7. The rotary encoder H₁ detects the angle of swing motion of the vertical-swing linkage including the links 9. The electromagnetic clutch C₁ and other electromagnetic clutches incorporated into the medical three-dimensional locating apparatus are disengaged when energized and are engaged mechanically by spring mechanisms when de-energized, respectively. Accordingly, even if the medical three-dimensional locating apparatus is disconnected accidentally from the power source due to trouble, such as power failure, the vertical-swing linkage including the links 9 and the associated parts remain locked in place to secure safety.

Parallel links 11a forming a first horizontal-swing linkage are joined pivotally for swing motion generally along the z-axis in a horizontal plane to one end of the vertical-swing linkage including the links 9. A counterweight W₁ including an instrument unit 12 is connected to the other end of the vertical-swing linkage including the links 9 to counterbalance the a gross weight acting on the parallel linkage of the links 9, so that the vertical-swing linkage including the links 9 can readily be operated without requiring large force. The first horizontal-swing linkage including the links 11a connected to the vertical-swing linkage opposite to the counterweight W₁ allows the support unit 3 transverse motions, namely, motions along the x-axis, in a horizontal plane. An electromagnetic clutch C₂ and a rotary encoder H₂ are connected to the joint of the vertical-swing linkage including the links 9 and the first horizontal-swing linkage including the links 11a.

A second horizontal-swing linkage formed of parallel links 11b pivotally supported on one end of the first horizontal-swing linkage including the links 11a for swing motion in a horizontal plane. The second horizontal-swing linkage including the links 11b allows the support unit 3 longitudinal motions, namely, motions along the z-axis, in a horizontal plane. An electromagnetic clutch C₃ and a rotary encoder H₃ are connected to the joint of the first horizontal-swing linkage including the links 11a and the second horizontal-swing linkage including the links 11b. The free end of the second horizontal-swing linkage including the links 11b is the extremity 14 of the support unit 3. The extremity 14 can be moved to a desired position by the combination of the vertical motions of the vertical-swing linkage including the links 9 and the horizontal and longitudinal motions of the first horizontal-swing linkage including the links 11a and the second horizontal-swing linkage including the links 11b.

The arm unit 2 is joined to the extremity 14 of the support unit 3. The arm unit 2 comprises a first arm 15 pivotally joined to the extremity 14 for turning motion about a horizontal first axis P₁, a vertical second arm 17 pivotally joined to the extremity 16 of the first arm 15 for turning motion about a second axis P₂ perpendicular to the first axis P₁, and an indicating unit 19 attached to the extremity 18 of the second arm 17, having directionality and position to become coaxial with the first axis P₁ and capable of moving toward and away from the intersection point S of the first axis P₁ and the second axis P₂. Counterweights W₂ and W₃ are connected to the respective base ends of the first arm 15 and the second arm 17 to counterbalance the gross weights acting on the first arm 15 and the second arm 17, respectively, so that the first arm 15 and the second arm 17 can readily be turned by a small force and can be stopped in an optional position. The indicating unit 19 attached to the extremity 18 of the second arm 17 can be moved along a spherical surface with its center at the intersection point S by the combined turning motion of the first arm 15 and the second arm 17. A pivot supporting the first arm 15 on the extremity 14 and a pivot supporting the second arm 17 on the first arm 15 are provided with an electromagnetic clutch C₄ and a rotary encoder H₄, and an electromagnetic clutch C₅ and a rotary encoder H₅, respectively. The electromagnetic clutches C₄ and C₅ fix the first arm 15 and the second arm 17, respectively. The rotary encoders H₄ and H₅ detects the respective angles θ and φ of turning of the first arm 15 and the second arm 17, respectively.

A straight rack 20 is attached to the extremity of the second arm 17. A holder 21 detachably engaging the rack 20 can be moved in opposite directions along the rack 20 by turning a knob 22. A holding member 21a of the holder 21 is swingable on a hinge 23. The cuboidal body 24 of the indicating unit 19 is positioned correctly on the holder 21 and is held firmly in place by the holding member 21a. When the cuboidal body 24 is held on the holder 21, the tip 25a of an indicating needle 25 attached to the cuboidal body 24 is directed always toward the intersection point S of the first axis P₁ and the second axis P₂. A rotary encoder H₆ is connected to the knob 22 for moving the holder 21 relative to the rack 20 to determine the distance l between the intersection point S of the first axis P₁ and the second axis P₂, and the tip 25a of the indicating needle 25 through the detection of the movement of the indicating unit 19. The holder 21 detachable from the rack 20, and the indicating unit 19 detachable from the holder 21 facilitate sterilizing the holder 21 and the indicating unit 19.

The electromagnetic clutches C₁ to C₅ and the rotary encoders H₁ to H₆ are connected to the instrument unit 12 mounted on the counterweight W₁. Provided on the panel of the instrument unit 12 are switches SW₁ to SW₅ for adjusting the positions of the intersection point S of the first axis P₁ and the second axis P₂ respectively on the y-axis, the x-axis and the z-axis, the angular position θ of the indicating needle 25 with respect to the intersection point S, and the angular position φ of the indicating needle 25 with respect to the intersection point S. When the switch SW₁ is closed, a current is supplied to the electromagnetic clutch C₁ to disengage the same. The electromagnetic clutches C₂, C₃, C₄ and C₅ are controlled similarly for engagement and disengagement by operating the switches SW₂, SW₃, SW₄ and SW₅, respectively. Accordingly, when only the switch SW₁, for instance, is ON and the rest of the switches SW₂ to SW₅ are OFF, only the electromagnetic clutch C₁ is disengaged and the rest of the clutches C₁ to C₅ remain engaged, so that the intersection point S in the arm unit 2 can be moved only in vertical directions. Thus, the switches SW₁ to SW₅ are controlled selectively to enable only the desired linkage or linkages among the linkages respectively including the links 9, 11a and 11b, and/or the desired arm or arms among the arms 15 and 17 to move.

Provided on the side surface of the second arm 17 are switches TS₁ and TS₂ and a data input button SB. the switches TS₁ and TS₂ are used for controlling the electromagnetic clutches C₄ and C₅. When the switch TS₁ is ON, power is supplied to the electromagnetic clutches among the electromagnetic clutches C₁ to C₅ excluding those corresponding to the switches which are OFF among the switches SW₁ to SW₅ of the instrument unit 12 to disengage the corresponding electromagnetic clutches among the electromagnetic clutches C₁ to C₅ of the support unit 3 in order that the support unit 3 can freely be moved. The position data of the tip 25a of the indicating needle 25 is entered by operating the data input button SB.

The computer MC is connected to the instrument unit 12. The computer MC analyzes data given thereto by the rotary encoders H₁ to H₆, and displays the results of analysis numerically on a display. The rotary encoders H₁ to H₆ are connected directly respectively to the corresponding rotary shafts of the links 9, 11a and 11b to reduce errors for accurate detection.

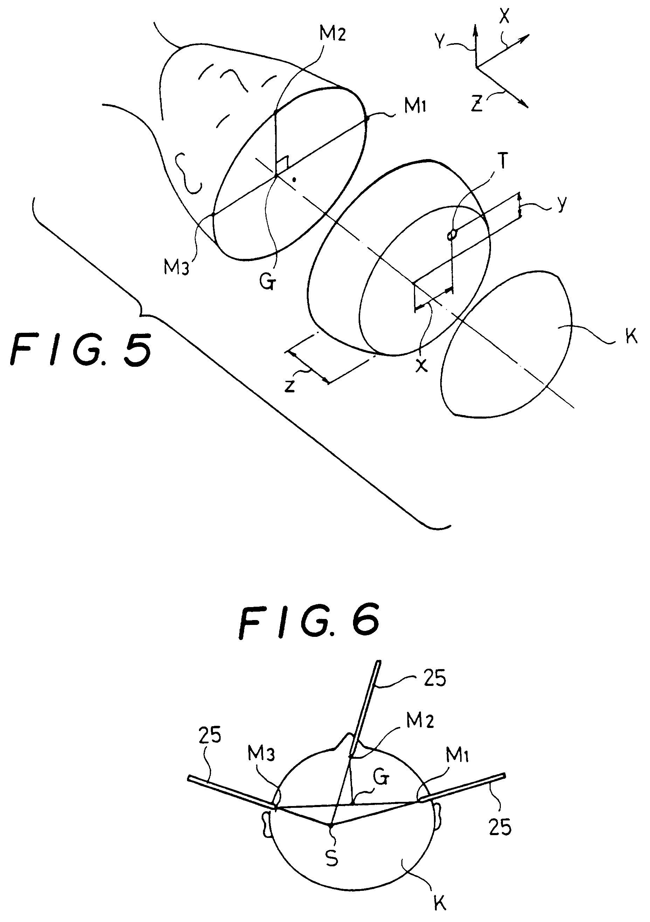

The operation of the medical three-dimensional locating apparatus will be described hereinafter. First, the patient's head K is held firmly by a fixing jig, and then three optional positions on the patient's head K are marked respectively with three marks M₁, M₂ and M₃. The number of positions on the patient's head K to be marked with marks need not be limited to three, but, when need be, four or more positions on the patient's head K may be marked with four or more marks. Small balls, not shown, of a material which can be detected by CT or MRI scanning, such as a metal, are secured by tapes to the patient's head K at the three positions marked respectively with the marks M₁, M₂ and M₃. Then, the patient's head K is set on a CT or MRI scanning apparatus for CT of MRI scanning to obtain the pictures of sections of the head K. Then, the origin G of the head K is determined with reference to the three marks M₁, M₂ and M₃ and, at the same time, the three-dimensional position data of the focus T with respect to the origin G. That is, the three-dimensional coordinates (x, y, z) or angles (θα, φα) at the origin G in the θ direction and the φ direction of the Focus T can be determined. As shown in Fig. 5, the origin G is the foot of the perpendicular from the mark M₂ on a straight line connecting the marks M₁ and M₃.

After the pictures have been taken by CT or MRI scanning and the origin G has been determined, the small balls attached to the head K at the positions marked with the three marks M₁, M₂ and M₃ are removed, and then the patient is transported with the head K held firmly by the fixing jig to an operating room. Since the head K is held immovable, the inclination of the head K with respect to a horizontal plane in the operating room is the same as that of the head K with respect to a horizontal plane (the floor) in which the head K was held during CT or MRI scanning. The patient's head K is set properly relative to the arm unit 2 of the medical three-dimensional locating apparatus 1. Then, the first arm 15 and the second arm 17 are turned to place the tip 25a of the indicating needle 25 sequentially on the marks M₁, M₂ and M₃ and the data input button SB is depressed to store data representing the positions of the marks M₁, M₂ and M₃ in the computer MC. Since the patient's head K is set arbitrarily relative to the arm unit 2, the intersection point S in which the tip 25a of the indicating needle 25 is directed and the origin G determined with reference to the marks M₁, M₂ and M₃ do not coincide with each other as shown in Fig. 6. The push-button of the switch TS₁ is depressed to disengage all the electromagnetic clutches C₁, C₂ and C₃ of the support unit 3, and then the arm unit 2 is moved vertically (along the y-axis), and horizontally (along the x-axis and the z-axis) to bring the intersection point S in the arm unit 2 into coincidence with the origin G in the head K.

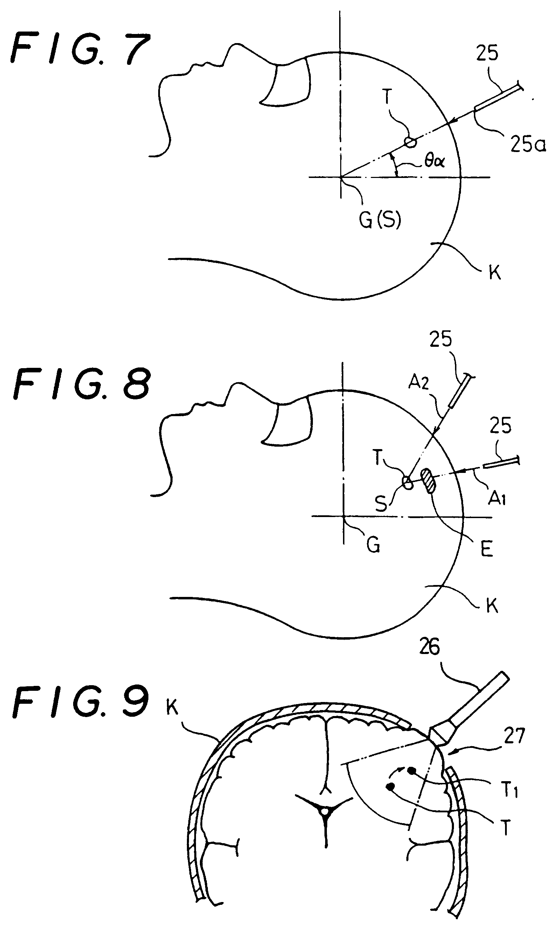

Then, the focus T is located. An angular focus locating method of locating the focus T on the basis of the angles (θα, φα) at the origin G respectively in the θ direction and the φ direction will briefly be described with reference to Fig. 7. The angles of the indicating needle 25 at the origin G with respect to the θ direction and the φ direction are displayed on the monitor screen of the computer MC. The arm unit 2 is operated to bring the angles of the indicating needle 25 into coincidence respectively with the angles (θα, φα) at the origin G with respect to the θ direction and the φ direction determined by using the picture obtained by CT or MRI scanning. In this state, the indicating needle 25 is directed toward the focus T. The angular focus locating method, however, is unable to readily change the approach angle with respect to the focus T. A coordinate focus locating method will be described with reference to Fig. 8.

The arm unit 2 is moved on the support unit 3 on the basis of the three-dimensional coordinates (x, y, z) of the focus T determined through CT or MRI scanning to shift the intersection point S in the arm unit 2 from the origin G to the focus T within the head K, in which the push-button of the switch TS₁ is depressed to disengage the electromagnetic clutches C₁, C₂ and C₃ of the support unit 3, and the arm unit 2 is moved on the support unit 3 so as to bring the present thee-dimensional coordinates of the intersection point S displayed on the monitor screen of the computer MC into coincidence with the coordinates (x, y, z) of the focus T determined through CT or MRI scanning.

Upon the coincidence of the intersection point S with the focus T, the indicating needle 25 is directed always to the focus T (intersection point S) regardless of the approach angle, and hence the focus T can surely be exposed through craniotomy along the direction indicated by the indicating needle 25. The depth of the focus T can be known from data provided by the encoder H₆ representing the positional relation between the tip 25a of the indicating needle 25 and the surface of the head K. Accordingly, an optional approach angle for reaching the focus T by surgical operation can readily be selected. For example, if the craniotomy is performed at an approach angle A₁, the focus T is at the shortest distance from the position for surgical opening on the skull. However, if important nervous tissues E or the like are situated between the position for surgical opening and the focus T as shown in Fig. 8, this position of surgical opening cannot be used because it is possible that the important nervous tissues E or the line are damaged if the craniotomy is performed at the approach angle A₁. In such a case, the craniotomy must be performed at an approach angle other than the approach angle A₁, for example, at an approach angle A₂. Reference to the pictures of the head obtained through CT or MRI scanning facilitates determining an optimum approach angle. Since the medical three-dimensional locating apparatus is unnecessary after the position for surgical opening of the skull has been determined, the pedal 8 of the support unit 3 is operated to turn the support unit 3 accurately through an angle of 180° on the post 7, and then craniotomy is performed at the thus determined position for surgical opening of the skull. When it is necessary to confirm the position of the focus T again after the operation has been started, the pedal 8 is operated again to turn the support unit 3 through an angle of 180° in the opposite direction to set the arm unit 2 accurately at the initial set position with respect to the patient's head K. Thus, the arm unit 2 can readily and accurately be located again at the initial set position for the reconfirmation of the position for surgical opening of the skull and other conditions.

In this embodiment, the intersection point S in the arm unit 2 is brought into coincidence with the origin G in the head K, and then the intersection point S is shifted from the origin G to the focus T. However, if the relation between the initial position of the intersection point S and the position of the focus T can be determined through analysis by the computer MC, the intersection point S may directly be brought into coincidence with the focus T.

The support unit 3 supporting the first arm 15 may be a known robot arm or a moving device linearly moving in the X-, the Y- and the Z-direction instead of the combinations of the linkages including the links 9, 11a and 11b.

The rotary encoders H₁ to H₆ may be substituted by potentiometers or suitable position detectors.

A medical three-dimensional locating apparatus in another embodiment which is not part of the present invention will be described hereinafter with reference to Fig 9, in which parts like or corresponding to those of the foregoing embodiment are denoted by the same reference characters and the description thereof will be omitted.

The medical three-dimensional locating apparatus in this embodiment employs an echo probe 26 instead of the indicating unit 19. The echo probe 26 is held on the holder 21 of the second arm 17. An echo picture is produced by ultrasonic waves emitted from the tip of the echo probe 26 and propagating in a sectorial region. The tip of the echo probe 26 is applied to an opening 27 formed in the head K to locate the focus T in case the focus T is caused to moved to a position T₁ by change in the internal pressure of the head after the opening 27 has been formed. Thus, the focus T can surely be located even if the focus T moves to the position T₁

A sensor, a therapeutic apparatus for burying an electrode in the head or for perforation biopsy or an optical apparatus, such as a microscope, may be held by the holder 21 on the second arm 17.

As is apparent from the foregoing description, the intersection point of the first and second axes in the arm unit of the medical three-dimensional locating apparatus in accordance with the present invention is fixed and the tip of the indicating unit is directed always toward the fixed intersecting point. Accordingly, the tip of the indicating unit is directed always toward a focus in the patient's head, once the intersection point in the arm unit is located at the focus by adjusting the position of the arm unit on the support unit, and hence an optimum approach angle (an optimum position for craniotomy) can readily be selected through a simple operation. Since the position of the intersection point of the first and second axes in the arm unit and the position of the tip of the indicating unit relative to the intersection point are detected by position detectors, the three-dimensional position data obtained through CT or MRI scanning can accurately be reproduced in the patient's head for surgical operation by bringing the data provided by the position detectors into coincidence with the three-dimensional position data of the target (focus) obtained through CT or MRI scanning, monitoring the position data provided by the position detectors and displayed on the monitor screen of the computer in digital data.

The present invention has the following effects.

Counterbalanced respectively with the counterweights, the vertical-swing linkage of the support unit and the arms can rapidly and safely be moved without requiring a large force. Since the support unit can accurately be turned through an angle of 180° when the pedal is operated, the arm unit can be moved away from the operative position to the inoperative position after determining the approach angle to avoid the arm unit interfering with the surgical operation, and the arm unit can accurately be located again at the operative position with the intersection point in the arm unit located accurately at the initial position where the intersection point was located before the arm unit was moved to the inoperative position.

The removable holder and the removable indicating unit facilitate gas-sterilization of the same.

## Claims

1. A medical three-dimensional locating apparatus comprising:
an arm unit (2) supported by a support unit (3) so that the arm unit (2) can optionally be moved vertically and horizontally in three perpendicular directions (X, Y and Z), said arm unit (2) comprising a first arm (15) pivotally supported by said support unit (3) for turning about a first axis (P₁) and a second arm (17) pivotally supported on said first arm (15) for turning about a second axis (P₂), said second axis (P₂) being perpendicular to and arranged to intersect said first axis (P₁), and an indicating unit (19) mounted on said second arm (17), characterised in that said indicating unit (19) is mounted on said second arm (17) so as to be movable along a spherical surface centred on the intersection point as a result of combined pivoting of the first arm (15) and the second arm (17) and so as to be capable during such movement of becoming coaxial with the first axis (P₁), and said indicating unit (19) further being directed towards the intersection point and being displaceable towards and away from the intersection point, and in that position detectors (H) are arranged to detect the position of the intersection point of the first and second axes (P₁, P₂) in the arm unit (2) and the position of a tip (25a) of the indicating unit (19) relative to the intersection point.

2. A medical three-dimensional locating apparatus according to claim 1 characterised in that the support unit (3) comprises a combination of vertical-swing parallel linkages (9, 11).

3. A medical three-dimensional locating apparatus according to claim 1 or 2, characterised in that the position detectors are encoders.

## Patentansprüche

1. Medizinische dreidimensionale Lokalisierungsvorrichtung, aufweisend:
eine von einer Stützeinheit (3) unterstützte Armeinheit (2) dergestalt, daß die Armeinheit (2) frei wählbar vertikal und horizontal in drei senkrecht aufeinanderstehenden Richtungen (X, Y, Z) bewegt werden kann, wobei die Armeinheit (2) einen schwenkbar von der Stützeinheit (3) gelagerten ersten Arm (15) zum Drehen um eine erste Achse (P₁) und einen schwenkbar an dem ersten Arm (15) gelagerten zweiten Arm (17) zum Drehen um eine zweite Achse (P₂) aufweist, wobei die zweite Achse (P₂) senkrecht zu der ersten Achse (P₁) steht und so angeordnet ist, daß sie die erste Achse (P₁) schneidet, und eine auf dem zweiten Arm (17) montierte Anzeigeeinheit (19), dadurch gekennzeichnet, daß die Anzeigeeinheit (19) auf dem zweiten Arm (17) so befestigt ist, daß sie beweglich ist entlang einer Kugelfläche, die ihren Mittelpunkt auf dem Schnittpunkt hat, als Ergebnis kombinierter Verschwenkung des ersten Arms (15) und des zweiten Arms (17), und daß sie während einer solchen Bewegung koaxial mit der ersten Achse (P₁) werden kann, und wobei die Anzeigeeinheit (19) zu dem Schnittpunkt hin ausgerichtet ist und zu dem Schnittpunkt hin und von diesem weg geschoben werden kann, und daß Positionsdetektoren (H) angeordnet sind, um die Position des Schnittpunktes der ersten und zweiten Achse (P₁, P₂) in der Armeinheit (2) und die Position einer Spitze (25a) der Anzeigeeinheit (19) relativ zu dem Schnittpunkt zu detektieren.

2. Medizinische dreidimensionale Lokalisierungsvorrichtung nach Anspruch 1, dadurch gekennzeichnet, daß die Stützeinheit (3) eine Kombination von Vertikalschwing-Parallelgestängen aufweist.

3. Medizinische dreidimensionale Lokalisierungsvorrichtung nach Anspruch 1 oder 2, dadurch gekennzeichnet, daß die Positionsdetektoren Kodierer sind.

## Revendications

1. Appareil médical de localisation tridimensionnelle comprenant :
un ensemble formant bras (2) supporté par un ensemble formant support (3) de telle sorte que l'ensemble formant bras (2) peut être éventuellement déplacé verticalement et horizontalement selon trois directions perpendiculaires (X, Y et Z), ledit ensemble formant bras (2) comprenant un premier bras (15) supporté pivotant par ledit ensemble formant support (3) pour tourner autour d'un premier axe (P₁) et un second bras (17) supporté pivotant sur ledit premier bras (15) pour tourner autour d'un second axe (P₂), ledit second axe (P₂) étant perpendiculaire au premier axe (P₁) et disposé de façon à lui être sécant, et un ensemble indicateur (19) monté sur ledit second bras (17), caractérisé en ce que ledit ensemble indicateur (19) est monté sur ledit second bras (17) de façon à être mobile le long d'une surface sphérique centrée sur le point d'intersection par suite du pivotement combiné du premier bras (15) et du second bras (17) et à être capable, pendant un tel mouvement, de devenir coaxial avec ledit premier axe (P₁), et ledit ensemble indicateur (19) étant en outre dirigé vers le point d'intersection et étant déplaçable vers le, et à l'écart du, point d'intersection et en ce que des détecteurs de position (H) sont disposés de façon à détecter la position dudit point d'intersection des premier et second axes (P₁, P₂) dans l'ensemble formant bras (2) et la position d'une pointe (25a) de l'ensemble indicateur (19) par rapport au point d'intersection.

2. Appareil médical de localisation tridimensionnelle selon la revendication 1, caractérisé en ce que l'ensemble formant support (3) comprend une combinaison de bielles parallèles à oscillation verticale (9, 11).

3. Appareil médical de localisation tridimensionnelle selon la revendication 1 ou 2, caractérisé en ce que les détecteurs de positions sont des codeurs.
